Europäisches Patentamt

European Patent Office

Office européen des brevets

(19)

(11) Publication number: **0 085 678**

**B1**

(12) **EUROPEAN PATENT SPECIFICATION**

(45) Date of publication of patent specification: **07.01.87**

(21) Application number: **82900676.6**

(22) Date of filing: **01.03.82**

(86) International application number:
**PCT/SE82/00057**

(87) International publication number:
**WO 82/03127 16.09.82 Gazette 82/22**

(51) Int. Cl.⁴: **G 01 N 31/22,** G 01 N 33/52,
C 12 Q 1/30, G 01 N 21/77

(54) **INDICATOR.**

(30) Priority: **02.03.81 SE 8101322**

(43) Date of publication of application:
**17.08.83 Bulletin 83/33**

(45) Publication of the grant of the patent:
**07.01.87 Bulletin 87/02**

(84) Designated Contracting States:
**AT BE CH DE FR GB LI NL**

(56) References cited:
**DE-A-2 729 333**
**DE-A-2 922 856**
**DE-A-2 934 760**
**FR-A-2 029 138**
**GB-A-1 526 708**
**US-A-3 723 064**
**US-A-3 964 871**

(73) Proprietor: **ALFA-LAVAL AB**
**Box 500**
**S-147 00 Tumba (SE)**

(72) Inventor: **BJÖRLING, Karl Torsten**
**Grödingevägen 65**
**S-147 00 Tumba (SE)**
Inventor: **GRÖNBERG, Ann-Marie Märta Skyle**
**Abbots Gardens, 93**
**London, N.2. (GB)**

(74) Representative: **Lerwill, John et al**
**A.A. Thornton & Co. Northumberland House**
**303-306 High Holborn**
**London, WC1V 7LE (GB)**

EP 0 085 678 B1

Courier Press, Leamington Spa, England.

## Description

This invention relates to an indicator for detecting at least one test substance in a test medium. More particularly the invention is concerned with an indicator for detecting the presence of a test substance in a test medium, comprising a carrier and a reaction system including a first reactant and a second reactant applied to respective areas of the carrier, the first reactant being capable, upon contact with said test medium, of releasing a mobile substance, which mobile substance is able to pass to the second reactant by diffusion through the carrier soaked in the test medium, and which mobile substance reacts with said test substance and with said second reactant.

An indicator of this form is disclosed in GB—A—1526708 which describes an indication e.g. for detecting of the enzyme catalase in milk. The reaction system employed consists partly of a first enzyme for generating hydrogen peroxide, namely glucose and the enzyme glucose oxidase, and partly of a color-generating system consisting of the enzyme peroxidase and o-tolidine, which latter is converted from a colorless state into a colored state, if it is allowed to react with hydrogen peroxide. In principle, the first, hydrogen peroxide-generating system is applied to a carrier spaced from the color-generating system. More especially the reactants are distributed uniformly over the carrier surface, either as dots or parallel stripes, so that there is equal spacing between each area portion of the carrier to which the color-generating system is applied and the nearest area of the carrier to which the hydrogen peroxide generating system is applied. When the test is performed, the carrier — preferably a paper strip — is soaked with the test medium, that is to say the milk. Hydrogen peroxide is evolved, which travels by diffusion in the direction towards the color-generating system. On its way the hydrogen peroxide has to travel by diffusion through a part of the carrier, which is soaked by test medium. If the latter contains the substance being detected, i.e. catalase, the latter will react with the hydrogen peroxide to an extent, that is determined by its concentration, and no or a limited quantity of the hydrogen peroxide generated reaches the color-generating system to give rise to a color change. The intensity of the color thus depends on the concentration of any remaining hydrogen peroxide and thus inversely on the concentration of catalase in the test medium.

This prior art indicator may give good results, but they are dependent on a number of factors. The accuracy of the catalase determination thus demands an exact time schedule for the test procedure, good light and a color scale for comparison between the color developed and the color scale, to provide the catalase concentration figures at different color tinges. The color tinge may show considerable variation due to variations in the color of the milk itself, disturbing substances in the milk or the air, and the age of the indicator. The color tinges in the color system peroxidase-o-tolidine may also vary with different charges in commercial production.

An indicator in which the reactants are applied to the carrier as a matrix of dots uniformly distributed over the carrier surface is also disclosed in DE—A—2729333, and this indicator suffers the same disadvantages as those described.

The present invention seeks to provide an indicator which alleviates the drawbacks mentioned above and an indicator in accordance with the invention is characterized in that the first reactant is confined to a limited area of the carrier, and some area portions of the carrier to which the second reactant is applied are farther than are other such area portions, from the respectively nearest area of the carrier to which the first reactant is applied.

By the reactants being applied to the carrier according to the invention the mobile substance, e.g. hydrogen peroxide, released by the first reactant must travel along diffusion paths of different lengths through the carrier to reach the second reactant, e.g. a color generating system like peroxidase-o-tolidine, and as a result will be consumed to a greater or smaller extent, according to the diffusion path length, by any of the test substance being detected present in the test medium in which the indicator is soaked. Thus, a longer path of diffusion gives a greater opportunity for a higher degree of reaction between the mobile substance and the test substance, which means that a correspondingly less amount of the mobile substance can reach the second reactant to form e.g. a color.

Consequently, the color pattern developed, rather than merely the intensity of color, is indicative of the concentration of the test substance in the test medium.

It is probably most convenient to use such reaction systems, which give rise directly to a visually detectable color change, even if other reaction systems could be considered, e.g. such systems, in which the color is only developed by spraying a developer on it.

Suitably the carrier of the indicator is made of a planar material, such as paper sheet or the like.

In one particular embodiment of the invention the first reactant is applied as a stripe on a surface of the carrier, and the second reactant is also applied as a stripe, distance between the stripes varying, preferably increasing continuously.

It is also for the first reactant to be applied to one side of the carrier and the second reactant to be applied on the other side of the carrier. An especially good effect is obtained if the first reactant is applied in the form of a stripe extending in a first direction, and the second reactant is applied as several substantially parallel stripes extending in a direction different, preferably perpendicular to said first direction.

In another embodiment of the invention the first reactant is applied onto one side of a carrier, within an area with a substantially circular periphery, and the second reactant is applied to the other side of the carrier within areas in the form of concentric mutual spaced apart zones. In use a distinct number of rings is colored/not colored.

The invention is not limited to any special mode of manufacture, but it is probably suitable to apply the reactants by printing technique, known per se.

The invention is described more in detail below with reference being made to the accompanying drawings, in which:—

Figure 1 shows in longitudinal section, and grossly enlarged an indicator in the form of a paper strip;

Figure 2 shows an indicator, provided with reactant one one side surface, seen from above;

Figure 3 shows an indicator provided with reactants on both sides, Figure 3A being a perspective view and Figures 3(b)—3(d) plan views; and

Figure 4 shows, in a similar way to Figure 3, another embodiment of an indicator with reactants on both sides.

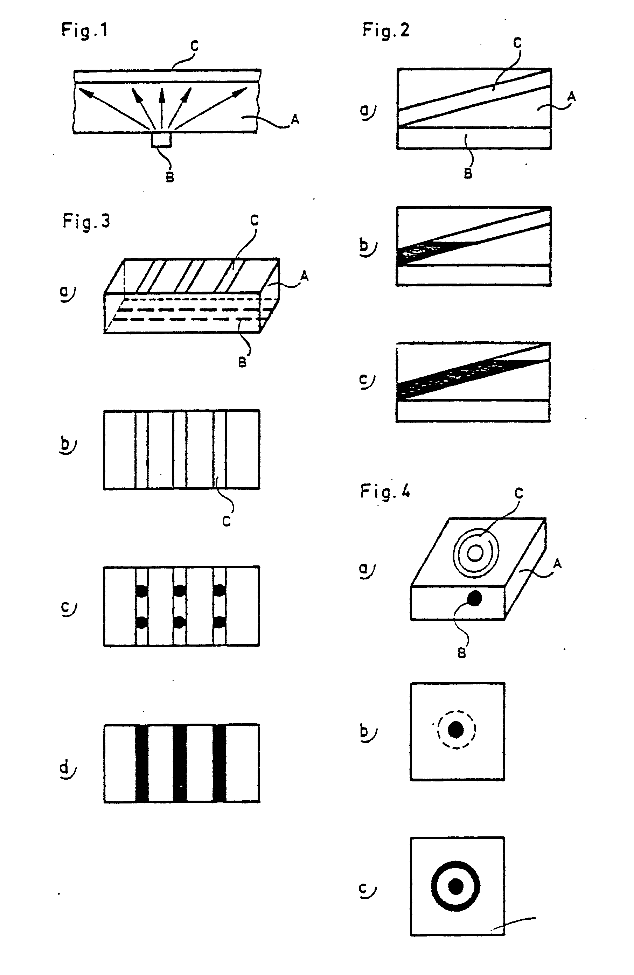

The indicator shown in Figure 1 consists of a paper strip on the lower side edge of which there has been applied a first reactant B, in the form of a transverse stripe and the upper side edge of which is coated with a second reactant C. When used, the complete strip is soaked in the test medium or sample, which is to be investigated, the carrier thus being impregnated with any test substance A to be detected and present in the sample. When reactant B is brought into contact with the test medium, usually a liquid, the reactant, or a mobile substance liberated from the reactant B, starts to travel by diffusion in the direction of the arrows shown. Thereby a certain amount of the mobile reactant will travel by diffusion straight to the upper side, whilst other amounts will have longer and longer paths of diffusion to reactant C. As the concentration of test substance A in the test medium increases, the chances for the mobile reactant from B to reach reactant C will decrease, and the longer diffusion path the greater opportunity for it to react with test substance A. This means that a relatively greater concentration of mobile reactant will reach reactant C applied to area portions of the carrier closest to the area of the carrier to which reactant B is applied, and a relatively low concentration will reach reactant C applied to area portions located further away. If C is a reactant, which will show a color directly when reacted with the mobile reactant B, this will means a correspondingly decreasing color tinge with increased diffusion path. Thus the color pattern obtained is indicative of the concentration of the test substance A in the test medium. The indicator in Figure 2 is provided with a stripe or band of reactant B and a stripe band of reactant C, the bands being inclined at an acute angle to each other. The indicator is shown in Figure 2a before use; in Figure 2b after use for the analysis of a test medium with a relatively high concentration of test substance A; and in Figure 2c after use for the analysis of a test medium with relatively low concentration of A. It will be seen that the color pattern obtained is indicative of the concentration of the test substance. The indicator shown in Figure 3 is provided on the lower side with reactant B applied as longitudinal stripes or bands and on the upper side with transverse stripes or bands of reactant C. The indicator is shown in Figure 3b after use for the analysis of a sample with a high concentration of test substance A; in Figure 3c after analysis of a sample with a moderately high concentration of test substance A; and in Figure 3d after analysis of a sample with a low concentration of test substance A. Once again it can be clearly seen that the color pattern produced depends on the concentration of the test substance.

Figure 4 shows an indicator, provided on the lower side with reactant B in the form of a small round spot, and on the upper side provided with a number of concentric rings of reactant C, spaced from each other. The indicator is shown in Figure 4a after the analysis of a sample with a relatively high concentration of test substance A; in Figure 4b after the analysis of a sample with a moderate concentration of test substance A, and in Figure 4c after analysis of a sample with a relatively low concentration of test substance. As for the preceding embodiment, the color pattern obtained indicates the concentration of the test substance.

The indicator according to the invention has the following advantages, as compared to indicators, which have been used previously for similar purposes:

1. The sensitivity of the indicator for a test substance can be varied within wide limits, by using carriers with adapted resistance to diffusion, e.g. in the form of paper sheets of suitable thickness.

2. The color scale can be printed in black/white as it is the color pattern rather than the color tinge that indicates the measuring result.

3. The reading of the indicator is simple and can be performed by non-skilled staff.

4. The reading result is relatively insensitive to factors like light, color of sample, disturbing components in the sample etc.

5. The time schedule for reading of the indicator is less critical, in practical cases within an interval of some minutes.

6. The indicator can be manufactured, advantageously, by conventional printing technique.

The indicator can be used for concentration measurement of a number of substances, especially enzymes. In the examples shown, the first reactant is e.g. glucose, applied separated from, but in the vicinity of glucose oxidase, and the other reactant is peroxidase mixed with o-tolidine. Such an indicator can be used for the measurement of the concentration of catalase in milk.

The indicator can also be used, advantageously, for the determination of other substances than enzymes. Thus an indicator for the determination of total acid concentration (expressed e.g. as mM $H^+$) can be designed according to the example just disclosed, with a reactant B consisting of an alkaline substance, like sodium hydroxide, and a reactant C consisting of a pH-indicator with a suitable detection interval, e.g. bromthymol blue for neutral pH. When soaking the indicator with test liquid, the alkaline substance will travel by diffusion through the test liquid reacting with any acid available, and any remaining alkali will be indicated by the reactant C.

**Claims**

1. An indicator for detecting the presence of a test substance (A) in a test medium, comprising a carrier and a reaction system including a first reactant (B) and a second reactant (C) applied to respective areas of the carrier, the first reactant (B) being capable, upon contact with said test medium, of releasing a mobile substance, which mobile substance is able to pass to the second reactant (C) by diffusion through the carrier soaked in the test medium, and which mobile substance reacts with said test substance (A) and with said second reactant (C), characterised in that the first reactant (B) is confined to a limited area of the carrier, and some area portions of the carrier to which the second reactant (C) is applied are farther than are other such area portions, from the respectively nearest area of the carrier to which the first reactant is applied.

2. An indicator according to claim 1, wherein the carrier is a substantially planar material, and the first and second reactants (B, C) are each applied to area in the form of a stripe on a surface of the carrier.

3. An indicator according to claim 1, wherein the carrier is a substantially planar material, the first reactant (B) is applied to one side of the carrier, and the second reactant (C) is applied to the other side of the carrier.

4. An indicator according to claim 3, wherein the first reactant (B) is applied to an area in the form of a stripe extending in a first direction, and the second reactant (C) is applied to areas formed by a plurality of substantially parallel stripes extending in a direction different to said first direction.

5. An indicator according to claim 3, wherein the first reactant (B) is applied to an area having a substantially circular periphery, and the second reactant is applied to areas in the form concentric, mutually spaced apart zones.

6. An indicator according to any of claims 2 to 5, wherein at least one reactant (B, C) is applied by printing technique, known per se.

**Revendications**

1. Indicateur destiné à détecter la présence d'une substance d'essai (A) dans un milieu d'essai, comprenant un support et un système réactionnel comprenant un premier réactif (B) et un second réactif (C) appliqués à des zones respectives du support, le premier réactif (B) pouvant, en entrant en contact avec le milieu d'essai, libérer une substance mobile, ladite substance mobile étant susceptible de parvenir au second réactif (C) par diffusion à travers le support immergé dans le milieu d'essai, et ladite substance mobile réagissant sur ladite substance d'essai (A) et sur le second réactif (C), caractérisé en ce que le premier réactif (B) est confiné dans une zone limitée du support, et en ce que certaines des portions du support auxquelles le second réactif (C) est appliqué sont plus éloignées que d'autres de ces portions de support de la zone respectivement la plus proche du support à laquelle le premier réactif est appliqué.

2. Indicateur selon la revendication 1, caractérisé en ce que le support est un matériau sensiblement plan, en ce que le premier réactif (B) et le second réactif (C) sont appliqués chacun à la zone sous la forme d'une bande sur une face du support.

3. Indicateur selon la revendication 1, caractérisé en ce que le support est un matériau sensiblement plan, en ce que le premier réactif (B) est appliqué à une face du support, et en ce que le second réactif (C) est appliqué à l'autre face du support.

4. Indicateur selon la revendication 3, caractérisé en ce que le premier réactif (B) est appliqué à une zone en forme de bande s'étendant dans une première direction, et en ce que le second réactif (C) est appliqué à des zones formées par une pluralité de bandes sensiblement parallèles s'étendant dans une direction différente de la première direction.

5. Indicateur selon la revendication 3, caractérisé en ce que le premier réactif (B) est appliqué à une zone présentant une périphérie sensiblement circulaire, et en ce que le second réactif est appliqué à des zones se présentant sous la forme de zones concentriques mutuellement séparées.

6. Indicateur selon l'une quelconque des revendications 2 à 5, caractérisé en ce qu'au moins un réactif (B, C) est appliqué par une technique d'impression, connue en soi.

**Patentansprüche**

1. Anzeigevorrichtung zum Ermitteln des Vorliegens einer Prüfsubstanz (A) in einem Prüfstoff mit einem Träger und einem Reaktionssystem aus einem ersten Reagens (B) und einem zweiten Reagens (C), die in entsprechenden Flächenteilen des Trägers aufgetragen sind, wobei das erste Reagens (B) bei der Berührung mit dem Prüfstoff eine bewegliche Substanz freisetzen kann, die durch Diffusion durch den mit dem Prüfstoff getränkten Träger zum zweiten Reagens (C) wandern kann und mit der Prüfsubstanz (A) und dem zweiten Reagens (C) reagiert, dadurch gekennzeichnet, daß das erste Reagens (B) auf einen begrenzten Flächenteil des Trägers beschränkt ist und einige der Flächenbereiche des Trägers, auf die das zweite Reagens (C) aufgetragen ist, weiter als andere Flächenbereiche von den jeweils nächstliegenden, mit dem ersten Reagens versehenen Flächenbereichen des Trägers entfernt sind.

2. Anzeigevorrichtung nach Anspruch 1, bei der der Träger ein im wesentlichen flächiges Material ist und das erste und das zweite Reagens (B, C) jeweils auf einen Flächenbereich in Form eines Streifens auf die Trägeroberfläche aufgetragen sind.

3. Anzeigevorrichtung nach Anspruch 1, bei der der Träger ein im wesentlichen flächiges Material ist und das erste Reagens (B) auf die eine Seite des Trägers und das zweite Reagens (C) auf die andere Seite des Trägers aufgetragen sind.

4. Anzeigevorrichtung nach Anspruch 3, bei der das erste Reagens (B) auf einen Flächenbereich in Form eines in einer ersten Richtung verlaufenden Streifens und das zweite Reagens (C) in Flächenbereichen aufgetragen sind, die von einer Vielzahl von im wesentlichen parallelen Streifen gebildet sind, die in einer von der ersten unterschiedlichen Richtung verlaufen.

5. Anzeigevorrichtung nach Anspruch 3, bei der das erste Reagens (B) in einem Flächenbereich mit im wesentlichen kreisrundem Umfang und das zweite Reagens in Flächenbereichen in Form beabstandeter konzentrischer Zonen aufgetragen sind.

6. Anzeigevorrichtung nach einem der Ansprüche 2 bis 5, bei der mindestens eines der Reagenzien (B, C) durch ein an sich bekanntes Druckverfahren aufgetragen ist.

Fig.1

Fig.2

a)

Fig.3

a)

b)

c)

b)

c)

Fig.4

a)

c)

d)

b)

c)

1